# EUROPÄISCHE PATENTSCHRIFT

(11) **EP 2 165 719 B1**
(45) Veröffentlichungstag und Bekanntmachung des Hinweises auf die Patenterteilung: **08.07.2015**
(21) Anmeldenummer: 09011349.9
(22) Anmeldetag: 04.09.2009
(51) Int. Cl.: A61L 26/00

(54) **Wundversorgungszubereitung mit verminderten Hautirritationen**
Wound care preparation with reduced skin irritations
Préparation de pansements avec irritations réduites de la peau

(30) Priorität: 22.09.2008 DE 102008048338
(43) Veröffentlichungstag der Anmeldung: 24.03.2010
(73) Patentinhaber: Beiersdorf AG, 20245 Hamburg (DE)
(72) Erfinder: Kummer, Andreas, B., Dr., 21079 Hamburg (DE); Heuer, Björn, Dr., 22145 Hamburg (DE)
(74) Vertreter: Hartmann, Jost

(56) Entgegenhaltungen:
- WO-A1-90/03809

## Beschreibung

Die Erfindung beschreibt eine Wundversorgungszubereitung, die auf der Haut keinen Brennen verursacht (no-sting), einfach mit verschiedenen filmbildenden Polymeren zu formulieren ist, eine gute Filmbildung zeigt und eine schnelle Trocknung des applizierten Film aufweist.

Bekannt sind filmbildende Polymerisate, die in gelöster Form zur Herstellung von Wundabdeckungen dienen können. Die Lösungen können als Aerosole aus einem Aerosolbehälter in dünnen Schichten auf eine zu behandelnde Schnitt-, Schürf- oder Operationswunde aufgesprüht oder durch Pinsel oder anderweitige Auftragsverfahren auf den Wundbereich appliziert werden. Nach Abdunsten des Lösungsmittels bildet sich auf der Wunde ein dünner, zusammenhängender Film aus, der die Verletzungsstelle abdeckt und den Wundbereich gegen äußere Einflüsse schützt.

Flüssige Verbandsmaterialien oder flüssige Pflaster werden als solche zusammen mit einem Treibmittel in Aerosolbehältern abgefüllt in den Handel gebracht, den sog. Wundverband-Sprays, aus denen sie bei Bedarf durch Betätigung des Ventilknopfes auf die Wunde aufgesprüht werden können. Bekannt sind Sprühpflastersysteme wie sie in den Patentschriften DE 2343923 und DE 2924042 beschrieben sind.

Beispielsweise Hansaplast^{®} Sprüh-Pflaster besteht im Wesentlichen aus einem filmbildenden Polymer auf Acrylatbasis, gelöst in einem Lösemittelgemisch (Ethylacetat), aus Treibmitteln (n-Pentan, CO2) und einem Zusatzstoff (Menthol) als zusätzlicher Geruchs- und Wirkstoff mit leicht desinfizierender Wirkung.

Da eine wesentliche Anforderung an die Wundabdeckung eine hohe Wasserfestigkeit ist, sind die Polymere zumeist in einem organischen Lösungsmittel gelöst. Dieses muss nach dem Aufsprühen möglichst schnell trocknen, um eine angenehme Abdeckung der Wunde in möglichst kurzer Zeit zu erreichen. Häufig wird hierfür Ethylacetat eingesetzt. Ethylacetat hat, wie viele andere organische Lösungsmittel auch, die Eigenschaft auf Wunden ein teilweise stark ausgeprägtes Brennen zu verursachen. Dies ist ein negativer Aspekt der Produkte, die auf diesen Lösungsmitteln basieren, speziell bei empfindlichen Anwendern wie z.B. Kindern.

Alternativen sind nur sehr wenige vorhanden, da die Anforderungen (Lösung des Polymers und schnelles Verdampfen) nur wenige organische Lösungsmittel erfüllen, die meistens ebenfalls ein Brennen auf Wunden verursachen. Eine Möglichkeit ist die Verwendung von Polydimethylsiloxanen, wie z.B. Hexamethyldisiloxan oder cyclischen Siloxanen.

Die EP 572416 und EP 438496 beschreiben Wundversorgungszubereitungen, die auf der Haut schnell trocknen, flexibel anwendbar, wasserundurchlässig und luftdurchlässig sind und nicht schmerzen. Sie umfassen Siloxan enthaltende Polymere, wie Copolymere aus Silanderivaten und Methacrylaten, und flüchtigen Silikonen, wie beispielweise Hexamethyldisiloxan. Die Gewichtsanteile des flüchtigen Silikons Hexamethyldisiloxan (HMDS) liegen dabei zwingend zwischen 60 und 99 Gew.%.

Nachteilig ist an Hexamethyldisiloxan insbesondere die Einschränkung in der Auswahl geeigneter Polymere, die sich mit HMDS lösen lassen. Zudem wäre es aus allgemeinen Gründen der Verminderung von Chemikalien wünschenswert auch den Anteil an HMDS zu senken.

WO 0226278 A2 beschreibt Hautschutzfilme auf Basis von Cycloalkylmethacrylat-Copolymeren. Die Filme umfassen neben flüchtigen Lösemitteln wie Hexamethyldisiloxan, Isododekan etc. zwingend einen Weichmacher, wie Acetyltributylcitrat, um überhaupt die geforderte Flexibilität des Films auf der Haut zu gewährleisten. Nachteilig ist, das niedermolekulare Weichmacher aus den Filmen in die Haut respektive Wunde penetrieren können. Somit sind Hautirritationen möglich. Silikonacrylat-Polymere werden als filmbildende Polymere ebenso wenig erwähnt wie die Möglichkeit der Aerosol-Sprühapplikation, wozu die in der WO 0226278 genannten Zubereitungen nicht fähig sind.

WO 0010540 A1 beschreibt als Aerosol versprühbare filmbildende Zubereitungen. Die Zubereitungen umfassen ein Polymersystem gebildet aus auf der Haut klebenden Acyraltpolymeren und nichtklebenden Polymeren, wie Silikonen. Beide Polymere müssen nicht mischbar sein um die geforderten Wundabdeckungseigenschaften zu erreichen. Silikonacrylat-Polymere werden als filmbildende Polymere nicht erwähnt. In den Zubereitungen sind darüber hinaus Lösemittelgemische, beispielsweise umfassend Hexamethyldisiloxan und Isopropylalkohol, enthalten.

Isopropylakohole führen auf der Haut aber bekanntlich zu einem Brennen (stinging), das erfindungsgemäß zu vermeiden ist.

Die bekannten flüssigen Wundzubereitungen haben zumeist Nachteile hinsichtlich Verlauf und Filmbildung, insbesondere aufgrund einer begrenzten maximalen Polymerkonzentration, welche nur recht dünne und daher die Wunde wenig schützende Filme ausbilden.

Aufgabe der vorliegenden Erfindung ist es ein Wundversorgungsmaterial bereit zu stellen, dass die Vorzüge einer schmerzfreien Anwendung (no-sting), einer einfachen Formulierbarkeit verschiedener Polymere, einer guten Filmbildung und einer schnellen Trocknung des applizierten Films in sich vereinigt.

Gelöst werden die Aufgaben durch eine Wundversorgungszubereitung umfassend ein oder mehrere Silikon-Acrylat Polymere und ein oder mehrere Silikonether.

Erfindungsgemäß ist der Anteil an Silikonethern auf weniger als 55 Gew.%, bezogen auf die Gesamtmasse der Zubereitung, begrenzt.

Zusätzlich ist erfindungsgemäß ein Trägermittel zugesetzt, das aus der Gruppe der bei Raumtemperatur gasförmigen oder flüssigen linearen, cyclischen oder verzweigten Alkanen oder Alkenen mit einer C-Anzahl von 3 bis 20 oder deren Mischungen, und/oder Ether, die sich vom Silikonether unterscheiden, gewählt werden kann.

Als Silikon-Acrylat Polymere können im Sinne der Erfindung Copolymere, Block-Copolymere, Pfropf-Copolymere oder Blends mit einer Komponente auf Basis von Acrylaten und einer Komponente auf Basis von Silikonen eingesetzt werden. Acrylate können im Sinne der Erfindung alle Polymere sein, die als ein Monomerbaustein Acrylsäure, Methacrylsäure und/oder deren Derivate, wie z.B. Methyl-, Ethyl-, Butyl-, tert-Butyl-, Iso-Butyl, Octyl-, Ethylhexyl- oder andere Alkylester der Acryl- oder Methacrylsäure enthalten. Weitere Monomere können substiutierte Alkylester der Acryl- oder Methacrylsäure, Styrol oder Styrolderivate, Vinyl- oder Allylverbindungen und alle anderen, mit Acrylsäure oder deren Derivaten copolymerisierbare Monomere sein. Silikone können im Sinne der Erfindung alle Verbindungen sein, die als Struktureinheit SiR'R"R '" O, SiR'R"0₂ und / oder SiR'0₃ aufweisen, wobei R', R" und R"' unabhängig voneinander für einen linearen, cyclischen oder verzweigten Alkylrest mit 1-50 C-Atomen steht. Die Silikone im Sinne der Erfindung können sowohl linear, cyclisch als auch verzweigt sein.

Besonders bevorzugt sind Silikone der Struktur R'Si(OR²)₃, wobei R' einen Rest bezeichnet, über den das Silikon an das Acrylat-Polymer gebunden werden kann und R² für den Rest -SiMe2-(C2H4)-Si(0-SiMe3)3 steht.

Die bekannten flüssigen Wundzubereitungen haben zumeist Nachteile hinsichtlich Verlauf und Filmbildung, insbesondere aufgrund einer begrenzten maximalen Polymerkonzentration, welche nur recht dünne und daher die Wunde wenig schützende Filme ausbilden.

WO 9003809 A1 offenbart flüssige filmbildende Wundversorgungszubereitung umfassend 1 bis 40 Gew.% eines siloxanhaltigen Polymers, 59,9 bis 98,9 Gew.% eines flüchtigen Polydimethylsiloxans und 0,01 bis 10 Gew.% eines polaren Lösemittels, wie Alkohole oder Ethylglykole.

Aufgabe der vorliegenden Erfindung ist es ein Wundversorgungsmaterial bereit zu stellen, dass die Vorzüge einer schmerzfreien Anwendung (no-sting), einer einfachen Formulierbarkeit verschiedener Polymere, einer guten Filmbildung und einer schnellen Trocknung des applizierten Films in sich vereinigt.

Gelöst werden die Aufgaben durch eine Wundversorgungszubereitung umfassend ein oder mehrere Silikon-Acrylat Polymere und ein oder mehrere Silikonether.

Erfindungsgemäß ist der Anteil an Silikonethern auf bis zu 55 Gew.%, bezogen auf die Gesamtmasse der Zubereitung, begrenzt.

Zusätzlich ist erfindungsgemäß ein Trägermittel zugesetzt, das aus der Gruppe der bei Raumtemperatur gasförmigen oder flüssigen linearen, cyclischen oder verzweigten Alkanen oder Alkenen mit einer C-Anzahl von 3 bis 20 oder deren Mischungen, und/oder Ether, die sich vom Silikonether unterscheiden, gewählt werden kann.

Als Silikon-Acrylat Polymere können im Sinne der Erfindung Copolymere, Block-Copolymere, Pfropf-Copolymere oder Blends mit einer Komponente auf Basis von Acrylaten und einer Komponente auf Basis von Silikonen eingesetzt werden. Acrylate können im Sinne der Erfindung alle Polymere sein, die als ein Monomerbaustein Acrylsäure, Methacrylsäure und/oder deren Derivate, wie z.B. Methyl-, Ethyl-, Butyl-, tert-Butyl-, Iso-Butyl, Octyl-, Ethylhexyl- oder andere Alkylester der Acryl- oder Methacrylsäure enthalten. Weitere Monomere können substiutierte Alkylester der Acryl- oder Methacrylsäure, Styrol oder Styrolderivate, Vinyl- oder Allylverbindungen und alle anderen, mit Acrylsäure oder deren Derivaten copolymerisierbare Monomere sein.

Silikone können im Sinne der Erfindung alle Verbindungen sein, die als Struktureinheit SiR'R"R"' O, SiR'R"0₂ und / oder SiR'0₃ aufweisen, wobei R', R" und R'" unabhängig voneinander für einen linearen, cyclischen oder verzweigten Alkylrest mit 1-50 C-Atomen steht. Die Silikone im Sinne der Erfindung können sowohl linear, cyclisch als auch verzweigt sein.

Besonders bevorzugt sind Silikone der Struktur R'Si(OR²)₃, wobei R' einen Rest bezeichnet, über den das Silikon an das Acrylat-Polymer gebunden werden kann und R² für den Rest -SiMe2-(C2H4)-Si(0-SiMe3)3 steht.

Ganz besonders bevorzugt sind Silikon-Acrylat Polymere der INCI Acrylates/ Polytrimethylsiloxymethacrylate Copolymer wie das FA 4002 ID Silikone Acrylate der Firma Dow Corning. Ebenfalls bevorzugt sind Silikon-Acrylat Polymere der INCI Acrylates/Dimethicone Copolymer wie das KP-550 von Shin-Etsu.

Der Anteil der Silikon/Acrylat Polymere beträgt bevorzugt 5 bis 25 Gew.%, bezogen auf die Gesamtmasse der Zubereitung.

Silikonether im Sinne der Erfindung sind alle bei Raumtemperatur und Normaldruck flüssigen linearen, cyclischen oder verzweigten Silikone mit der Struktureinheit -SiR₂0-, wobei R für einen linearen, cyclischen oder verzweigten Alkylrest mit 1-50 C-Atomen steht. Besonders bevorzugt sind Polydimethylsiloxane wie Hexamethyldisiloxan (HMDS).

Der Anteil an HMDS beträgt im Stand der Technik 60% und mehr. Eine Reduzierung des Anteils an HMDS auf weniger als 60% hat daher offensichtlich Einbussen hinsichtlich der Filmbildung, Trocknungsgeschwindigkeit und Applikation der Zubereitung zur Folge.

Erfindungsgemäß ist es nun erstmals möglich den Anteil an Silikonethern auf weniger als 60 Gew.%, bezogen auf die Gesamtmasse der Zubereitung, zu begrenzen.

Bevorzugt liegt der Anteil an Silikonethern, insbesondere HMDS, im Bereich 30 bis 55 Gew.%, auf die Gesamtmasse der Zubereitung. Vorteilhaft ist der Anteil an Silikonethern, insbesondere HMDS, kleiner als 50 Gew.%.

Erfindungsgemäß ist die Reduzierung an HMDS auf einen Anteil von weniger als 56% bzw. 50% möglich indem der Zubereitung ein Trägermittel zugesetzt wird.

Als erfindungsgemäße Trägermittel wirken bevorzugt Alkan oder Alkan-Gemische. Im Sinne der Erfindung kann jedes bei Raumtemperatur und Normaldruck flüssige oder gasförmige lineare, cyclische oder verzweigte Alkan, Alken oder Isoalkan, mit einer C-Anzahl von 3 bis 20 bzw. Mischungen aus diesen eingesetzt werden. Besonders geeignet sind gasförmige Alkane wie Propan, Butan und/oder Isobutan, die unter Druck flüssig werden und daher in Aerosoldosen für einen konstanten Druck sorgen und sich darüber hinaus auch als Treibgase eignen. Besonders geeignet sind die flüssigen Alkane oder Isoalkane als Trägermittel um damit die Zubereitung als Sprühpflaster zu verwenden.

Bevorzugt ist auch der Einsatz von Isododekan als Trägermittel.

Auch können Ether als erfindungsgemäße Träger eingesetzt werden. Ether im Sinne der Erfindung kann jeder bei Raumtemperatur und Normaldruck flüssige oder gasförmige Ether der allgemeinen Strukturformel R-O-R' sein, wobei R und R' voneinander unabhängig für lineare, cyclische oder verzweigte Kohlenwasserstoffe mit 1-50 C-Atomen stehen. Besonders geeignet sind gasförmige Ether wie Dimethylether, die unter Druck flüssig werden und daher in Aerosoldosen für einen konstanten Druck sorgen, sich somit auch besonders gut als Treibgase eignen.

Die als Trägermittel erfindungsgemäß einsetzbaren Ether sind nicht identisch mit den als Silikonether definierten Verbindungen.

Besonders bevorzugt als Trägermittel sind die Kombinationen aus Isododekan und Dimethylether, Isosdodekan und einer Mischung aus Propan, Butan und/oder Isobutan oder Isododekan, Dimethylether und einem bei Raumtemperatur gasförmigen Alkan.

Die Kombination aus Acrylates/ Polytrimethylsiloxymethacrylate Copolymer und/oder Acrylates/Dimethicone Copolymer als Silikon-Acrylat Polymere, HMDS zu einem Anteil von weniger als 56 Gew% sowie ein oder mehrere Trägermittel gewählt aus der Gruppe Propan, Butan, Isobutan, Isododekan und/oder Dimethylether führt zu einer Wundversorgungszubereitung, die sprühbar ist, schnell trocknet und kein Brennen auf der Haut oder Wunde verursacht. Auf den Zusatz an Ethylacetat kann vollständig verzichtet werden.

Ebenso kann auf den Zusatz von Weichmachern, insbesondere Citratweichmacher, wie Acetyltributylcitrat, verzichtet werden.

Bevorzugt sind als Trägermittel Isododekan und des weiteren Dimethylether, Propan, Butan und/oder Isobutan.

Mit der Erfindung werden erstmals die Vorzüge einer schmerzfreien Anwendung (no-sting), eine einfache Formulierbarkeit verschiedener Polymere mit einer guten Filmbildung und einer schnellen Trocknung des applizierten Films in einer einzigen Zubereitung bereit gestellt. Die Formulierung zeichnet sich weiterhin durch die Eignung für eine Anwendung als Aerosol aus, was eine einfache und hygienische Applikation des Wundversorgungsproduktes auf der Haut ermöglicht.

Durch die Erfindung sind Formulierungen mit hohen Anteilen Polymer zugänglich, die eine optimale Trocknungskinetik und Filmbildung aufweisen, die sich zudem leicht versprühen lassen. Vergleichbare Formeln, die einen Anteil an HMDS von mehr als 60% aufweisen, liefern in der Regel einen schlechter haftenden Film und verlaufen nach Applikation leichter auf der Haut.

Der erfindungsgemäße Wundverband lässt sich vorteilhaft auf die Haut aufsprühen. Die Zubereitung ist ein sprühbares Wundversogungskit, welches einen wasserfesten, beständigen und elastischen Film bildet und innerhalb kürzester Zeit auf der Haut trocknet. Wesentlich ist, dass dabei kein unangenehmes Brennen auf der Haut oder der Wunde verspürt wird.

Zusätzlich können Wirkstoffe dem Sprühpflaster zugesetzt sein, wie solche mit kühlenden, schmerzlindernden, pflegenden und/oder bakteriziden, viruziden und/oder pflegenden Eigenschaften.

Die eingesetzten Wirkstoffe können sowohl systemisch als auch lokal wirksam sein. Bevorzugt eingesetzte Wirkstoffe sind hierbei:
Ätherische Öle, wie Menthol, Aceclidin, Amfetaminil, Amfetamin, Amylnitrit, Apophedrin, Atebrin, Alpostadil, Azulen, Arecolin, Anethol, Amylenhydrat, Acetylcholin, Acridin, Adenosintriphosphorsäure, L-Äpfelsäure, Alimemazin, Allithiamin, Allyl Isothiocyanat, Aminoethanol, Apyzin, Apiol, Azatadin, Alprenolol, Äthinazon, Benzoylperoxid, Benzylalkohol, Bisabolol, Bisnorephe¬drin, Butacetoluid, Benactyzin, Campher, Colecalciferol, Chloralhydrat, Clemastin, Chlorobutanol, Capsaicin, Cyclopentamin, Clobutinol, Chamazulen, Dimethocain, Codein, Chloropromazin, Chinin, Chlorthymol, Cyclophosphamid, Cinchocain, Chlorambuzil, Chlorphenesin, Diethylethan, Divinylethan, Dexchlorpheniramin, Dinoproston, Dixyrazin, Ephedrin, Ethosuximid, Enallylpropymal, Emylcamat, Erytroltetranitrat, Emetin, Enfluran, Eucalyptol, Etofenamat, Ethylmorphin, Fentanyl, Fluanison, Flurbiprofen, Guajazulen, Halothan, Hyoscyamin, Histamin, Fencarbamid, Hydroxycain, Hexylresorcin, Isoaminilcitrat, Isosorbiddinitrat, Ibuprofen, Jod, Jodoform, Isoaminil, Lidocain, Lopirin, Levamisol, Methadon, Methyprylon, Methylphenidat, Mephenesin, Methylephedrin, Meclastin, Methopromazin, Mesuximid, Nicethamid, Norpseudoephedrin, Menthol, Methoxyfluran, Methylpentinol, Metixen, Mesoprostol, Oxytetracain, Oxyprenolol, Oxyphenbutazon, Oxychinolin, Pinen, Prolintan, Procyclidin, Piperazin, Pivazid, Phensuximid, Procain, Phenindamin, Promethazin, Pentetrazol, Profenamin, Perazin, Phenol, Pethidin, Pilocarpin, Prenylamin, Phenoxybenzamin, Resochin, Scopolamin, Salicylsäure, Salicylsäureester, Spartein, Trichlorethylen, Timolol, Trifluperazin, Tetracain, Trimipramin, Tranylcypromin, Trimethadion, Tybamat, Thymol, Thioridazin, Valproinsäure, Verapamil, sowie weitere, dem Fachmann geläufige, über die Haut, eingeschlossen Schleimhäute, aufnehmbare Wirkstoffe. Selbstverständlich ist diese Aufzählung nicht abschließend.

Bevorzugt ist das erfindungsgemäße Wundversorgungskit als Sprühpflaster formuliert, so dass neben den erfindungsgemäßen Bestandteilen noch weitere Stoffe, wie Parfume zugegen sein können.

Als Aerosol, in Quetschflaschen oder in Treibgasflaschen ist eine Anwendung möglich und bevorzugt, besonders bevorzugt ist die Aerosolform.

Auch eine Pinselapplikation der erfindungsgemäßen Zubereitung ist erfindungsgemäß.

Nachfolgende Beispiele illustrieren einige erfindungsgemäße Wundversorgungszubereitungen. Die angegebenen Anteile sind Gewichtsanteile bezogen auf die Gesamtmasse der Zubereitungen.

### Darin bedeutet

Polymer 1: Silikon-Acrylat Polymer Dow Corning FA 4002 ID und
Polymer 2: Silikon-Acrylat Polymer KP-550 von Shin Etsu

### Beispiel 1:

| | |
|---|---|
| Polymer 1 | 10 |
| | |
| Isododecan | 15 |
| Hexamethyldisiloxan | 55 |
| Propan/Butan/Isobutan | 20 |

### Beispiel 2:

| | |
|---|---|
| Polymer 1 | 15 |
| | |
| Isododecan | 22,5 |
| Hexamethyldisiloxan | 42,5 |
| Propan/Butan/Isobutan | 20 |

### Beispiel 3:

| | |
|---|---|
| Polymer 1 | 20 |
| | |
| Isododecan | 30 |
| Hexamethyldisiloxan | 30 |
| Propan/Butan/Isobutan | 20 |

### Bespiel 4:

| | |
|---|---|
| Polymer 1 | 10 |
| | |
| Isododecan | 15 |
| Hexamethyldisiloxan | 55 |
| Dimethylether | 20 |

### Beispiel 5:

| | |
|---|---|
| Polymer 1 | 15 |
| | |
| Isododecan | 22,5 |
| Hexamethyldisiloxan | 42,5 |
| Dimethylether | 20 |

### Beispiel 6:

| | |
|---|---|
| Polymer 1 | 20 |
| | |
| Isododecan | 30 |
| Hexamethyldisiloxan | 30 |
| Dimethylether | 20 |

### Beispiel 7:

| | |
|---|---|
| Polymer 2 | 10 |
| | |
| Isododecan | 15 |
| Hexamethyldisiloxan | 55 |
| Propan/Butan/Isobutan | 20 |

### Beispiel 8:

| | |
|---|---|
| Polymer 2 | 15 |
| | |
| Isododecan | 22.5 |
| Hexamethyldisiloxan | 42,5 |
| Propan/Butan/Isobutan | 20 |

### Beispiel 9:

| | |
|---|---|
| Polymer 2 | 20 |
| Isododecan | 30 |
| Hexamethyldisiloxan | 30 |
| Propan/Butan/Isobutan | 20 |

### Beispiel 10:

| | |
|---|---|
| Polymer 2 | 10 |
| Isododecan | 15 |
| Hexamethyldisiloxan | 55 |
| Dimethylether | 20 |

### Beispiel 11:

| | |
|---|---|
| Polymer 2 | 15 |
| Isododecan | 22,5 |
| Hexamethyldisiloxan | 42,5 |
| Dimethylether | 20 |

### Beispiel 12:

| | |
|---|---|
| Polymer 2 | 20 |
| Isododecan | 30 |
| Hexamethyldisiloxan | 30 |
| Dimethylether | 20 |

### Beispiel 13:

| | |
|---|---|
| Polymer 1 | 5 |
| Polymer 2 | 5 |
| Isododecan | 15 |
| Hexamethyldisiloxan | 55 |
| Propan/Butan/Isobutan | 20 |

### Beispiel 14:

| | |
|---|---|
| Polymer 1 | 7,5 |
| Polymer 2 | 7,5 |
| Isododecan | 22,5 |
| Hexamethyldisiloxan | 42,5 |
| Propan/Butan/Isobutan | 20 |

### Beispiel 15:

| | |
|---|---|
| Polymer 1 | 10 |
| Polymer 2 | 10 |
| Isododecan | 30 |
| Hexamethyldisiloxan | 30 |
| Propan/Butan/Isobutan | 20 |

### Beispiel 16:

| | |
|---|---|
| Polymer 1 | 5 |
| Polymer 2 | 5 |
| Isododecan | 15 |
| Hexamethyldisiloxan | 55 |
| Propan/Butan/Isobutan | 20 |

### Beispiel 17:

| | |
|---|---|
| Polymer 1 | 7,5 |
| Polymer 2 | 7,5 |
| Isododecan | 22,5 |
| Hexamethyldisiloxan | 42,5 |
| Propan/Butan/Isobutan | 20 |

### Beispiel 18:

| | |
|---|---|
| Polymer 1 | 10 |
| Polymer 2 | 10 |
| Isododecan | 30 |
| Hexamethyldisiloxan | 30 |
| Propan/Butan/Isobutan | 20 |

Alle Formulierungen liefern nach dem Aufsprühen einen wasserfesten, beständigen, elastischen Film, trocknen innerhalb von einer Minute und verursachen auf frischen Wunden praktisch kein Brennen.

## Patentansprüche

1. Wundversorgungszubereitung umfassend ein oder mehrere Silikon-Acrylat Polymere und ein oder mehrere Silikonether, **dadurch gekennzeichnet, dass** der Anteil an Silikonethern bis zu 55 Gew.%, bezogen auf die Gesamtmasse der Zubereitung, beträgt und ein oder mehrere Trägermittel, gewählt aus der Gruppe der bei Raumtemperatur gasförmigen oder flüssigen, linearen, cyclischen oder verzweigten Alkanen oder Alkenen mit einer C-Anzahl von 3 bis 20 oder deren Mischungen, und/oder Ether, die sich vom Silikonether unterscheiden, in der Zubereitung enthalten sind.

2. Wundversorgungszubereitung nach Anspruch 1 **dadurch gekennzeichnet, dass** als Silikon-Acrylat Polymere Polymere gewählt werden, indem die Silikone die Struktur R'Si(OR²)₃ aufweisen, wobei R' einen Rest bezeichnet, über den das Silikon an das Acrylat-Polymer gebunden werden kann und R² für den Rest -SiMe2-(C2H4)-Si(0-SiMe3)3 steht.

3. Wundversorgungszubereitung nach Anspruch 1, **dadurch gekennzeichnet, dass** als Silikon-Acrylat Polymere Polytrimethylsiloxymethacrylate Copolymere und/oder Acrylates/Dimethicone Copolymere gewählt werden.

4. Wundversorgungszubereitung nach Anspruch 1, 2 oder 3, **dadurch gekennzeichnet, dass** als bei Raumtemperatur gasförmige Alkane Propan, Butan und/oder Isobutan gewählt werden.

5. Wundversorgungszubereitung nach einem der vorstehenden Ansprüche umfassend als Trägermittel Isododekan und des weiteren Dimethylether, Propan, Butan und/oder Isobutan.

6. Wundversorgungszubereitung nach einem der vorstehenden Ansprüche, **dadurch gekennzeichnet, dass** die Zubereitung weniger als 50 Gew.% Silikonether, bezogen auf die Gesamtmasse der Zubereitung, umfasst.

7. Wundversorgungszubereitung nach einem der vorstehenden Ansprüche in einer sprühbaren Form.

8. Wundversorgungszubereitung nach Anspruch 7 als Aerosolzubereitung.

9. Wundversorgungszubereitung nach einem der vorstehenden Ansprüche, **dadurch gekennzeichnet, dass** die Zubereitung kein Ethylacetat und/oder Weichmacher enthält.

## Claims

1. Wound care preparation comprising one or more silicone acrylate polymers and one or more silicone ethers, **characterized in that** the fraction of silicone ethers is up to 55% by weight, based on the total mass of the preparation, and one or more carriers selected from the group of linear, cyclic or branched alkanes or alkenes having a carbon number from 3 to 20 and are gaseous or liquid at room temperature, or mixtures thereof, and/or ethers which differ from the silicone ether are present in the preparation.

2. Wound care preparation according to Claim 1, **characterized in that**, as silicone acrylate polymers, polymers are selected by the silicones having the structure R'Si(OR²)₃, wherein R' designates a radical via which the silicone can be bound to the acrylate polymer, and R² is the radical -SiMe₂-(C2H4)-Si(0-SiMe3)₃.

3. Wound care preparation according to Claim 1, **characterized in that**, as silicone acrylate polymers, polytrimethylsiloxy methacrylate copolymers and/or acrylate/dimethicone copolymers are selected.

4. Wound care preparation according to Claim 1, 2 or 3, **characterized in that**, as alkanes gaseous at room temperature, propane, butane and/or isobutane are selected.

5. Wound care preparation according to any one of the preceding claims, comprising as carrier, isododecane and, in addition, dimethyl ether, propane, butane and/or isobutane.

6. Wound care preparation according to any one of the preceding claims, **characterized in that** the preparation comprises less than 50% by weight of silicone ether, based on the total mass of the preparation.

7. Wound care preparation according to any one of the preceding claims in a sprayable form.

8. Wound care preparation according to Claim 7 as aerosol preparation.

9. Wound care preparation according to any one of the preceding claims, **characterized in that** the preparation contains no ethyl acetate and/or plasticiser.

## Revendications

1. Préparation de soin des blessures comprenant un ou plusieurs polymères silicone-acrylate et un ou plusieurs éthers de silicone, **caractérisée en ce que** la proportion d'éthers de silicone est de jusqu'à 55 % en poids, par rapport à la masse totale de la préparation, et un ou plusieurs véhicules, choisis dans le groupe des alcanes ou alcènes linéaires, cycliques ou ramifiés, gazeux ou liquides à température ambiante, ayant un nombre de C de 3 à 20, ou leurs mélanges, et/ou des éthers différents de l'éther de silicone sont contenus dans la préparation.

2. Préparation de soin des blessures selon la revendication 1, **caractérisée en ce que** des polymères dans lesquels les silicones présentent la structure R'Si(OR²)₃, R' désignant un radical par lequel la silicone peut être reliée au polymère d'acrylate et R² représentant le radical -SiMe₂-(C₂H₄)-Si(O-SiMe₃)₃ sont choisis en tant que polymères silicone-acrylate.

3. Préparation de soin des blessures selon la revendication 1, **caractérisée en ce que** des copolymères de polytriméthylsiloxyméthacrylate et/ou des copolymères d'acrylate/diméthicone sont choisis en tant que polymères silicone-acrylate.

4. Préparation de soin des blessures selon la revendication 1, 2 ou 3, **caractérisée en ce que** le propane, le butane et/ou l'isobutane sont choisis en tant qu'alcanes gazeux à température ambiante.

5. Préparation de soin des blessures selon l'une quelconque des revendications précédentes, comprenant en tant que véhicule de l'isododécane et également de l'éther diméthylique, du propane, du butane et/ou de l'isobutane.

6. Préparation de soin des blessures selon l'une quelconque des revendications précédentes, **caractérisée en ce que** la préparation comprend moins de 50 % en poids d'éthers de silicone, par rapport à la masse totale de la préparation.

7. Préparation de soin des blessures selon l'une quelconque des revendications précédentes, sous une forme pulvérisable.

8. Préparation de soin des blessures selon la revendication 7, en tant que préparation d'aérosol.

9. Préparation de soin des blessures selon l'une quelconque des revendications précédentes, **caractérisée en ce que** la préparation ne contient pas d'acétate d'éthyle et/ou de plastifiant.
